# EUROPEAN PATENT SPECIFICATION

(11) **EP 4 643 772 B1**
(45) Date of publication and mention of the grant of the patent: **02.12.2026**
(21) Application number: 24192022.2
(22) Date of filing: 31.07.2024
(51) Int. Cl.: A61B 5/11, A61B 5/00

(54) **DYNAMIC POSTURE MONITORING SYSTEM WITH RECALIBRATION FEATURE FOR ARDUINO SENSOR INFORMATION**
DYNAMISCHES HALTUNGSÜBERWACHUNGSSYSTEM MIT REKALIBRIERUNGSFUNKTION FÜR ARDUINOSENSORINFORMATIONEN
SYSTÈME DE SURVEILLANCE DE POSTURE DYNAMIQUE AVEC CARACTÉRISTIQUE DE RÉÉTALONNAGE POUR INFORMATIONS DE CAPTEUR D'ARDUINO

(30) Priority: 30.04.2024 US 202418650216
(43) Date of publication of application: 05.11.2025
(73) Proprietor: BackAware Belt Ltd., Kilkenny R95 XP63 (IE)
(72) Inventor: Everard, Eoin, Kilkenny, R95 ARR7 (IE)

(56) References cited:
- CA-A1- 2 965 139
- GB-A- 2 592 665
- US-A1- 2015 065 919
- US-A1- 2023 233 104
- RIBEIRO PEDRO ET AL: "Spine Cop: Posture Correction Monitor and Assistant", SENSORS, vol. 20, no. 18, 19 September 2020 (2020-09-19), CH, pages 5376, XP093215733, ISSN: 1424-8220, DOI: 10.3390/s20185376

## Description

### TECHNICAL FIELD

The present application generally relates to a sensor system for measuring and evaluating the spinal position of an individual and detecting deviation from their desired position or exercise technique and receiving feedback for the same. More particularly, the present application relates to a system and method for monitoring and displaying user's spinal position on a mobile application or screen with a unique re-calibration feature allowing the user to set a desired position dynamically as per individual preferences. In this way they can use the disclosure for any lift or exercise and in any position they desire.

### BACKGROUND

It is well known that improper posture and exercise technique leads to muscular fatigue or more serious defects including disc herniations or repetitive stress injuries (RSI). Poor exercise technique and posture can have a detrimental effect on back health leading to issues such as bulging discs or low back pain. However, it can be difficult for people to understand when they move out of desired exercise positions. Moreover, unlike standing posture, the ideal position is not a static one. The most optimal position of the spine for a plank or press up type exercise is different to that required for squats or lunges.

Devices which are designed to detect poor posture and alert a user when such posture is detected are known in the art. US 4007733 A is one such example, which is a posture training device having adjustable shoulder straps connected to opposite points of a waistband of clothing of a user ad including signaling means to apply a sensory stimulus to the user and means to detect the relaxation of tension in the strap.

US 5199940 A is a postural training and correcting device which comprises a rod which is held firmly to a wearer's spine by way of adjustable elastic belts around the abdominal, shoulder, and head areas and achieves correct postural alignment by encouraging the wearer to hold in the abdominal muscles, hold the upper thoracic spine upright, and keep the neck and head in correct postural alignment with the wearer's body.

However, the prior art disclosures focus on highlighting deviations from an ideal or straight position i.e., the current devices provide a detection system for poor posture. But none of the devices address dynamic position calibration based on user preferences and varying postures. The above devices and other previous art would not be appropriate to use for various type of exercises on the ground, such as bridges or planks. Moreover, if the user actively wanted to keep a different type of posture it would not allow this. For example, strongmen lifting heavy boulders try to keep a flexed spine but do not wish to move from this position.

GB 2 592 665 A relates to a smart monitoring apparatus for spinal position, configured as a belt with integrated sensors to detect excessive flexion or extension of the spine. An electronic control unit processes the sensor data and provides feedback through vibration or Bluetooth notifications, thereby enhancing awareness of spinal alignment. The apparatus is suitable for use in occupational, fitness, and rehabilitation settings to reduce injury risk and support proper posture.

US 2023/233104 A1 relates to methods and systems for capturing and visualizing spinal motion using a wearable array of at least four capacitive stretch sensors arranged in an X-shaped configuration across the back (from shoulders to iliac spines). Sensor signals are processed to compute 3D spinal angular positions and to drive a personalized 3D spine model for visual biofeedback, optionally incorporating subject-specific parameters obtained by non-invasive scanning and using transfer functions that map sensor - array distortions to multi-axis angles.

The non-patent literature RIBEIRO PEDRO ET AL: "Spine Cop: Posture Correction Monitor and Assistant", SENSORS, vol. 20, no. 18, 19 September 2020 (2020-09-19), page 5376, XP093215733, CH ISSN: 1424-8220, DOI: 10.3390/s20185376 relates to a wearable posture correction and monitoring system, which integrates accelerometer, gyroscope, and magnetometer sensors with permanent magnets to assess spinal alignment in real time. Sensor data are processed by a probabilistic classification algorithm, calibrated for each user, to distinguish between correct and incorrect posture, with results indicating improved accuracy when combining accelerometer and magnetometer inputs. The device provides continuous feedback and posture scoring for therapeutic, ergonomic, and personal applications.

CA 2 965 139 A1 discloses a posture improvement device, system, and method. The disclosure combines a wearable sensor device, one or more user devices, and posture improvement software. The system identifies and monitors an individual's optimum postural positions (OPP) through sensors such as accelerometers and gyroscopes, providing real-time feedback, warnings, and reminders. Notifications may include visual, auditory, or tactile signals, and in some embodiments temporary disabling of user devices to enforce correction. The system further supports conditioning programs with stretching, exercise, and behavioral modification components to gradually improve posture and train neuromuscular awareness.

### OBJECT OF THE INVENTION

The object of the invention was therefore to eliminate the disadvantages of the prior art and to provide a dynamic posture monitoring system which can be used for various type of exercises.

### DISCLOSURE OF THE INVENTION

The object of the invention is solved by the features of the independent claims. Advantageous embodiments of the invention are described in the dependent claims.

In a first aspect, the application relates to a dynamic posture monitoring system which comprises a plurality of position sensors, an electronic control unit, a mobile application and a body attachment means. The dynamic posture monitoring system is in other words in particular a back position monitoring device with an associated app to monitor and display user's real-time spinal position. The advantage of the proposed system lies particularly in its ability to be used dynamically for different exercises without any changes to the arrangement of the system. This is particularly supported by the features described below.

In this regard, the position sensors and the control unit are integrated into the body attachment means. This allows the position sensors and the control unit to be worn around the user's body. This is particularly necessary to ensure a compact device that can accurately capture the position of the user's spine through the position sensors and, at the same time, includes a component in the control unit that can process the acquired data of the position sensors and/or transmit it to other devices such as a smart device.

The position sensors are preferably configured to monitor (excess) extension, flexion and movement of a user's spine in real time. The integration of a plurality of position sensors with real-time monitoring capabilities enhances the accuracy and responsiveness of the system in detecting spinal misalignment, thereby facilitating immediate corrective action.

The electronic control unit comprises a data processing unit, a vibration actuator and a Bluetooth module. The components of the electronic control are preferably supplied by a battery and/or a solar module.

The vibration actuator is configured to preferably notify and alert the user of a poor position of the spine by vibrating the body attachment means. The incorporation of a vibration actuator directly into the body attachment means provides an intuitive and discreet alert mechanism that can effectively prompt the user to adjust their posture without requiring visual or auditory cues.

The Bluetooth module is configured to preferably communicate with a smart device having a mobile application for real time visualization of user's spinal position, the mobile application further allowing a re-calibration of the position sensors depending on the user's desired spinal position. The Bluetooth module enables seamless wireless communication with a smart device, allowing for convenient real-time visualization and monitoring of the user's spinal position through a mobile application, which may encourage user engagement and adherence to proper posture practices.

Furthermore, the system allows for re-calibration of the position sensors based on the user's desired spinal position. This refers in particular to the process of adjusting a baseline or a reference point of the position sensors to match the specific posture that the user wants to maintain. In the context of the posture monitoring system, this means that users or an expert can set a new ideal spinal position for different activities or exercises. It is also possible that the new ideal spinal position is set automatically. The exact technical implementation of re-calibration or calibration is described below.

In the context of the described posture monitoring system, "dynamic" means preferably that the system can monitor and correct the user's posture in real-time and modify or adapt the ideal position of spinal position depending on the users desired position (or possibly automatically based on predetermined factors), regardless of the user's movements or activities. This is preferably achieved through the integration of multiple position sensors and an electronic control unit that work together to continuously capture the position of the spine and provide immediate feedback when necessary, and in particular through the user's smart device with the mobile application that allows for the recalibration of the position sensors depending on the user's desired spinal position (or possibly automatically based on predetermined factors). The user can re-calibrate or reset the zero value of the position sensors whenever needed, enabling them to set a new ideal posture or spinal position tailored to specific exercises. The dynamic nature of the system allows the user to wear the device during different exercises or daily activities without needing to readjust or change the arrangement of the system. This enhances the usability and effectiveness of the system, as it can seamlessly integrate into daily life and consistently provide accurate data.

Preferably, the data processing unit comprises a processor and a memory. The data processing unit is in data communication with the position sensors, the vibration actuator and the Bluetooth module. In particular, the data processing unit receives the acquired data from the position sensors as input data, processes this input data, and transmits output data to the Bluetooth module and/or as a control signal to the vibration actuator. The control signal for the vibration actuator causes the vibration actuator to vibrate when the data processing unit detects a bad or poor position of the user's spine.

A vibration actuator is preferably a component or a device that generates vibrations to provide haptic feedback to the user. Examples of vibration actuators include Eccentric Rotating Mass (ERM) motors, which use an off-center mass to create vibrations; Linear Resonant Actuators (LRA), which utilize a moving mass and electromagnetic coil for precise feedback; Piezoelectric actuators, which generate vibrations through the deformation of piezoelectric materials when an electric field is applied; and Voice Coil Actuators, which operate through the interaction of a magnetic field and electric current. These actuators can be seamlessly integrated into wearable body attachment means to provide effective haptic feedback.

A smart device is preferably an electronic device that is typically connected to other devices or networks via various wireless protocols such as Bluetooth, Wi-Fi, or cellular networks, and can operate interactively and autonomously. In the context of the posture monitoring system, a smart device is typically a smartphone or tablet that may run a mobile application designed to interface with the monitoring system. The smart device may comprise a data processing unit, a communication unit and a memory.

A poor position of the spine refers preferably to any posture that deviates from the natural, healthy alignment of the spine, which can lead to discomfort, strain, or long-term health issues. The poor position of the spine might include excessive slouching, overextension, flexion, or any misalignment that puts undue stress on the spinal column. These poor positions vary depending on the activity or situation. For example, a poor position while standing can differ from that while sitting, performing squats, lifting weights, doing sit-ups, or practicing yoga.

Therefore, it is essential to redefine what constitutes an optimal and non-optimal spine position before each activity.

The proposed system continuously calculates a value that represents the spine's posture using an algorithm that takes input from position sensors. The system allows for resetting and storing a representative value that indicates the spine's optimal position (re-calibration), for example, by a user or an expert. Based on this optimal position, upper and lower tolerance thresholds can be established (flexion and extension barriers, upper and lower limits), for example, by a user or an expert. Exceeding these thresholds with the continuously recorded value representing the spine's posture indicates that the spine is in a poor position.

The mobile application, commonly referred to as a mobile app, is a software application or computer program product designed to be installed or executed on a computer or smart device, such as a smartphone or tablet.

The plurality of position sensors may comprise a flex sensor, a strain sensor or any other spine position and displacement measuring sensor. Other possible sensors may include gyroscopes, accelerometers, Inertial Measurement Units (IMUs), magnetometers, pressure sensors, optical sensors, capacitive sensors, ultrasonic sensors, and electromagnetic tracking sensors. The inclusion of various types of position sensors, such as flex sensors and strain sensors, enables a comprehensive assessment of spinal position and displacement and covers a wide range of movements and postural deviations. The system's ability to utilize different sensor technologies ensures versatility and adaptability in monitoring the complex dynamics of spinal movement, which can be beneficial for users with varying physical conditions and postural needs.

The plurality of position sensors may comprise Arduino Flex Sensors, also known as bend sensors. These components measure the amount of bending or deflection by changing their resistance as they bend; the more they bend, the higher the resistance. This kind of flex sensors are often used in various electronics projects, such as game controllers, musical instruments, and robot controls.

To use a flex sensor with an Arduino (which is an open-source electronic platform which comprises a microcontroller or printed circuit board), a voltage divider circuit is commonly employed. This involves connecting the flex sensor and a fixed resistor in series, with the Arduino reading the voltage change that occurs as the sensor bends. The analog-to-digital converter (ADC) on the Arduino measures this voltage and interprets it to determine the degree of bending

The position sensors are integrated into the body attachment means in a way that the position sensors come in contact with the spine of the user. The strategic integration of position sensors into the body attachment means to contact the user's spine ensures direct and precise measurement of spinal alignment, leading to more accurate posture monitoring. By having sensors in close proximity to the spine, the system can detect even minor deviations in posture.

Furthermore, the body attachment means comprise a belt, clothing (not part of the invention), or any body attachment apparatus (not part of the invention). The provision of different body attachment means, such as a belt or clothing, offers flexibility in how the device can be worn, enhancing user comfort and ensuring consistent usage over extended periods. Other possible body attachment apparatus (not part of the invention) include a vest, which for example houses the sensors across the back and shoulders for comprehensive posture monitoring; a brace, providing for example additional support and ensuring sensor placement; adjustable straps worn for example over clothing; a harness for example to secure sensor placement during high-intensity activities; specially designed underwear for example for discreet monitoring; adhesive patches applied directly to the skin; a lightweight armature or exoskeleton; a specially designed backpack with integrated sensors; and arm or leg sleeves that incorporate sensors to monitor posture and related movements. These various body attachment apparatuses enhance the flexibility and usability of the posture monitoring system, catering to different preferences and activity levels.

The data processing unit is configured to receive data from the position sensors, to determine a poor position of the user's spine and to alert the user via the vibration actuator or by sending notification over the mobile application within the smart device of the user. The data processing unit's capability to analyze sensor data and determine poor spinal positions allows for automated and objective assessment of the user's posture, minimizing the need for manual input or monitoring. The option to receive alerts through either the vibration actuator or mobile application notifications provides users with flexible and personalized ways to be informed about their posture, accommodating different environments and user preferences.

The data processing unit employs algorithms to determine the poor position of the user's spine by receiving a multitude of values from multiple position sensors. For example, it may apply dimensionality reduction techniques (e.g., Principal Component Analysis (PCA), t-SNE, or autoencoders) to transform the high-dimensional sensor data into a lower-dimensional space. This transformation helps capture the essential features of the spinal position while reducing noise and computational complexity. The reduced-dimensional data can then be combined to create a simplified yet comprehensive representation of the current spinal position. The data processing unit may evaluate whether the current spinal position exceeds predefined thresholds. If the deviation exceeds these thresholds, a control signal may be generated and sent to activate the vibration actuator, providing immediate haptic feedback to the user. Optionally, a notification may be sent to the mobile application with detailed posture information and corrective suggestions. Upon receiving a recalibration request from the user via the mobile application or automatically, the lower-dimensional baseline data is updated with the new desired spinal position, and the thresholds and comparison metrics are adjusted accordingly.

For example, the recalibration may be triggered automatically when the system recognizes that the user is moving from one exercise to the next. The optimal spine position changes dynamically during an exercise so that the user selects a program before starting an exercise that automatically changes the desired spine position during the exercise. The mobile application may facilitate displaying spine position (or posture) through a digital sensor ball and digital flexion and extension barriers. The integration of a mobile application with a digital sensor ball enhances user engagement by providing a visual representation of the spine's position. The digital flexion and extension barriers serve as interactive guides that help users maintain their spine within a safe range of motion.

In this regard, the smart device may comprise a screen or may be connected to one. The smart device runs preferably a mobile application installed on it, which outputs to the screen. Specifically, a graphical element representing the current posture of the spinal position can be displayed. This graphical element is preferably a sensor ball, which is in particular a circular element. However, it can also take other shapes, such as a square, a rectangle, a polygon, or an avatar. The position of the sensor ball or graphical element can move across the screen as the user's current spinal position changes. The sensor ball can directly reflect the live values processed by the data processing unit of the control unit.

In addition to the sensor ball, the previously described thresholds can also be displayed on the screen. These are understood as digital flexion and extension barriers. For example, the thresholds can be represented as vertical stationary lines on the screen. The sensor ball moves in relation to these lines depending on the user's spinal posture, as detected by the position sensors and calculated by the data processing unit of the control unit. The sensor ball preferably moves in a horizontal direction. In the initial position, the sensor ball is located between the two threshold lines. If a poor position occurs, such as due to flexion of the back, the sensor ball may cross the left line; if an extension occurs, it may cross the right line.

In other words, the flexion and extension barriers can be seen as upper and lower limits respectively alerting the user of excessive movement. Alerting the user of excessive movement through these barriers can prevent overextension or overflexion. The graphical representation of the thresholds and the current posture of the spinal position allows the user to intuitively understand their posture and maintain the correct spinal position with ease.

In a further preferred embodiment, the flexion and extension barriers return to predetermined distances from the desired position upon selecting the re-calibration. The ability to return flexion and extension barriers to predetermined distances from the desired position upon re-calibration ensures that the system can adapt to the specific exercise or activity. Furthermore, the re-calibration feature allows for personalized adjustments to the system, accommodating different body types and flexibility levels, which enhances the system's usability across a diverse user base.

The user can set which exercise they want to perform and/or what the optimal posture of the spinal position should be. This is preferably done via the smart device, which is in data communication with the data processing unit. Based on the set optimal posture of the spinal position, the thresholds, i.e., the flexion and extension barriers, are set at a predefined distance from the digital sensor ball, representing the optimal position. If the user wants to perform a different exercise, they can adjust the optimal position accordingly. This can be done manually via the smart device or by positioning their body in the desired optimal posture for the exercise and then recalibrating the flexion and extension barriers. During recalibration, the flexion and extension barriers, represented by vertical lines, are reset at a predefined distance from the digital sensor ball, which has been moved to the new optimal position by the user. This new optimal position becomes the new baseline for the ideal posture.

It is also possible for the user, an expert or automatically to set a desired sensitivity of the position sensor movements depending on the user's spinal position. This can be done in two ways: software-based sensitivity adjustment and/or hardware-based sensor adjustment. For example, an algorithm will only take sensor data into account if it exceeds or falls below a threshold, where the threshold can be set. For hardware-based sensor adjustment, resistors can be set to physically adapt the sensitivity, or different types of sensors (which can be integrated) can be used that have different sensitivities depending on the user's movement or position.

In a further preferred embodiment, the user can visual the real time spinal movement in virtual reality by connecting the smart device with a virtual reality headset. Visualizing real-time spinal movement in virtual reality by connecting a smart device with a virtual reality headset offers an immersive experience that can significantly improve the user's understanding and awareness of their posture. The virtual reality feature can be particularly advantageously for educational and therapeutic purposes, as it allows for a more engaging and interactive approach to posture correction and training.

In a further aspect, a method of monitoring and displaying user's spinal position is provided. The method comprises the step of providing a dynamic posture monitoring system according to any of the preceding embodiments, wherein a user is wearing the body attachment means. Further, the method comprises the steps of:
- acquiring extension, flexion and movement of the user's spine in real time by the position sensor,
- detecting a poor position of the user's spine by the electronic control unit,
- communicating the acquired data of the position sensors and/or the detection of the user's poor spine position to a smart device of the user by the Bluetooth module and
- visualizing of the user's spinal position through the mobile application on the smart device and providing vibrations to the user through the vibration actuator.

The skilled person will recognize that the advantages, technical effects and preferred embodiments discussed in connection with the dynamic posture monitoring system apply analogously to the method of monitoring and displaying user's spinal position. Likewise, all the advantages, technical effects and preferred embodiments described in connection with the method are transferable to the dynamic posture monitoring system.

The method of monitoring and displaying the user's spinal position in real-time enables immediate correction of posture, which can lead to a reduction in back pain and the development of better postural habits. Providing vibrations to the user through the vibration actuator serves as a tactile alert for posture adjustment, which can be more effective than visual or auditory alerts, especially in noisy or visually distracting environments. The use of a smart device for visualizing the user's spinal position ensures that the system is portable and accessible, allowing users to monitor and improve their posture during various daily activities, In particular exercise technique.

The method may further comprise the step of re-calibrating the position sensors depending on the user's desired spinal position. As already mentioned above, the inclusion of a re-calibration feature ensures that the digital sensor ball is always aligned with the user's ideal position. This helps the user to be aware of their optimal position over time.

The method and/or posture monitoring system may allow for automatic re-calibration or resetting so that the method/system can adapt to changes in the user's posture over time, providing a personalized and dynamic posture monitoring system. For example, if the user is performing an exercise that may require different optimal positions of the back/spine during an exercise, such as the "cat-cow" exercise in yoga (where the back/spine is rounded (flexion) in a first position and arched (extension) in a second position), the optimal position may be updated / re-calibrated in the system over time. It's possible for the user to adopt an optimal posture for the exercise in both positions without having to adjust anything in the posture monitoring system, when the re-calibration step is automatic.

The mobile application facilitates visualization of spinal movement through a digital sensor ball and flexion and extension barriers, enhancing the user's understanding of their posture and/or spinal position during exercise technique.

The re-calibration may reset a digital sensor ball to the center of a digital graph based on the user's ideal position or desired spinal position. The graph here may preferably refer to a representation that shows an area that includes the digital sensor ball, the flexion and extension barriers. It also includes areas beyond the flexion and extension barriers so that poor positions can be easily displayed when the digital sensor ball has exceeded the flexion and extension barriers.

The re-calibration process ensures that the digital representation of the user's posture remains centered and accurate, which is important for precise monitoring and feedback. By resetting the digital sensor ball to the center of the digital graph, the method minimizes errors due to sensor drift or displacement, maintaining the integrity of the posture tracking system. For example, with a bend sensor, the measurement inaccuracy will be greater if the sensor is highly bend from an initial baseline. When a new baseline is set, the inaccuracy can decrease because the difference between a baseline and the off-axis value is less.

The digital graph is preferably a visual interface displayed on the smart device, providing a clear and intuitive representation of the user's spinal posture. This graph may for example include a central axis representing the ideal spinal position and additional markers or lines indicating the flexion and extension barriers. The digital sensor ball, which moves in real-time based on the user's spinal position, helps users quickly understand their current posture relative to the ideal position and any deviations that might indicate poor posture.

By utilizing this digital graph, users can easily track their posture during various activities and exercises. The interface allows for immediate visual feedback, which, combined with the tactile feedback from the vibration actuator, enhances the effectiveness of posture and/or spinal position (for example during exercise technique) correction. The re-calibration feature ensures that the digital graph remains accurate and personalized, adapting to the user's specific posture goals and exercise routines.

Based on the re-calibrated position, digital flexion and extension barriers of the mobile application may return to predetermined distance from the desired position. The adjustment of digital flexion and extension barriers ensures that the mobile application consistently provides feedback based on the user's desired posture.

In a further aspect, the application relates to a computer program product for determining an optimal posture of a user's spine. The computer program product comprises computer-executable instructions that are stored on a non-transitory computer-readable medium and that, when executed by a processor, cause an apparatus to:
- receiving real time data of plurality of position sensors which represent extension, flexion and movement of a user's spine;
- processing of received real time data by transformation into a single real time value, in particular through dimension reduction;
- defining of an upper and lower threshold and comparison with the real time value;
- displaying of the upper and lower thresholds by a line each and the real time value by a circle, wherein the lines of the upper and lower thresholds are parallel aligned, and the circle is positioned in relation to the lines, the circle positioned between both lines representing optimal posture of a user's spine.

The computer program product is preferably deployable on the smart device as described in the context of the dynamic posture monitoring system and the method of monitoring and displaying the user's spinal position. This program simplifies the interpretation of complex spinal movements and sensor data, making it easier for users to maintain proper spinal posture. Such a computer program product has not been previously known.

In particular, the transformation of complex sensor data into a single real-time value simplifies understanding the user's spinal posture, allowing users to easily interpret and respond to feedback. The visual representation of posture through lines and a circle provides an intuitive interface, enabling users to quickly assess and adjust their posture for optimal alignment.

In a further preferred embodiment, the computer program causes an apparatus further to:
- receiving an input of the user,
- determining a new upper and lower threshold depending on the user's desired spinal position,
- displaying the new upper and lower thresholds and setting the circle of the real time value in relation to the new upper and lower thresholds.

Alternatively, the dynamic posture monitoring system can be described as follows:
A dynamic posture monitoring system to monitor spine position, wherein the system includes two flex sensors integrated into the belt like apparatus that is capable of being positioned along the spine of the user to detect the flexed and extended position of the spine for a predefined or threshold period of time that may cause damage and injury to the wearer. These positions of excessive flexion and extension have been attributed to increased chance of injury leading to potential disability. To overcome this issue, the flex sensors are configured within the electronic control unit configured along with the belt that subsequently sends a signal to a vibration sensors situated within the control unit which intermediately vibrates to aware the user, when the spine of the wearer is in bad position.

The real time data of the wearer's spinal position is communicated and displayed on mobile application either on TV or within the smart device of the user making the user aware of the bad position. An electronic controller is also configured within the electronic unit of belt which is communicatively coupled with the flex monitoring sensors, vibration sensor, and with the mobile application within the smart device of user through a Bluetooth module.

Furthermore, the mobile application of the present application offers a re-calibration feature through a sensor ball on the screen, allowing users to customize the system based on their individual preferences and comfort levels. The coordination between the sensors, processing unit, vibration motor, and mobile application creates a synergistic relationship, ensuring precise spinal movement capture, effective feedback delivery, and user-friendly visualization. This comprehensive integration of components enables the system to provide a sophisticated and user-centric solution for spinal posture monitoring.

Another object of the present dynamic posture monitoring system is to allow the user to have an awareness of the position of their spine which is capable of being used for training purposes and proper positioning of the spine. Also, the present system may be used during the fitness and strength exercises to allows the user to set their preferred position as a good position and to be aware about their spinal position when upper and lower desired limits crosses the threshold limits and to help the user or exerciser with using correct technique being achieved on exercises and reduction in risk of injury that may be caused due to activation of wrong muscles by poor position of spine during exercise.

The present application further includes the ability to display the wearer and their spine position in the Meta Verse or Augmented/Virtual Reality through using the BackAware Belt and Arduino Sensors. A visual representation of the person can be seen in Meta Verse through an "avatar". The "avatar" will bend forward when the user bends in real life and the user can visually see the spine and can change the posture/correct the posture immediately. Furthermore, this system is compatible with other body parts and can provide real time information including but not limited to heart rate, spinal position for a holistic representation during exercise, and adaptability for integration into weightlifting or back support devices to offer feedback and additional support.

According to a preferred embodiment, the system further includes a Bluetooth module within the electronic unit of belt and communicatively coupled with the electronic controller of the belt. The Bluetooth module is configured to allow the Arduino sensors to communicate with the mobile application within the smart device of the wearer such as smart phone or any other smart wearable device and also to allow the user to monitor the real time position of their spine digitally using their smart devices. According to another embodiment of the present system, the Bluetooth module of the present system also allows the wearer or user to communicate with the Arduino sensors integrated within the belt, track flexion and extension during the day, visualize the position of the spine using the mobile application within the smart device of the user.

According to a preferred embodiment, the vibration sensor is further configured within the electronic unit of the belt that alert the wearer about excessive flexion or extension of their spine so that they may change the position of the spine. According to the present embodiment, the belt further comprises an electronic controller within the electronic unit, communicatively coupled with all the other components and modules of the present system and placed along with the vibrating sensor within the electronic unit. The electronic controller of the present application, receives the real time monitoring data from the Arduino sensors of the electronic unit, determines the poor position of the spine of the wearer and commands the vibration sensor present within the electronic unit of the dynamic posture monitoring system of present invention to alert the wearer about the poor position using vibration. Moreover, the electronic controller of the present system alerts the wearer about the poor position by also transmitting the notification over the smart devices of the wearer such as smart phone or any other wearable smart device along with the vibration on the belt.

According to a preferred embodiment, the invention can be helpful in a setting where employers can monitor and provide real time feedback on employee's back position. The ability to track the position can allow employers to send information to workers about their back positions and to make sure they are trying to keep good positions.

According to another preferred embodiment, the user or the wearer can see their spinal position in the Meta Verse or Augmented/Virtual Reality. The Arduino Flex sensors through Bluetooth communication module sends real-time spinal data over the virtual reality headset/helmet of the user. The visuals created in Meta Verse through "avatar" helps the user to correct the posture as the user's spine in real life can be visually seen in the headset. According to one more embodiment, which is not a part of the invention, instead of the belt, the user may use other clothing or body attachments to the skin for enhanced accuracy which can make it compatible with other body information such as heart rate for an overall user information during exercise or training. For example the flex sensors could be place in a t-shirt or compression garment.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation.

Further examples of embodiments are explained in more detail below with reference to the accompanying drawings.

### BRIEF DESCRIPTION OF THE FIGURES

- **Fig. 1A**: shows one exemplary embodiment of the dynamic posture monitoring system where a user is standing straight, and a screen of the user's smart device is shown.
- **Fig. 1B**: shows a front view of the belt worn by the user in Fig. 1A according to one embodiment of the present application.
- **Fig. 1C**: shows a back view of the belt worn by the user in Fig. 1A according to one more embodiment of the application.
- **Fig. 2**: shows another exemplary embodiment of the dynamic posture monitoring system, showing a user with hands and knees on the floor and a screen of the user's smart device.
- **Fig. 3**: shows another exemplary embodiment of the dynamic posture monitoring system, showing a user with hands and knees on the floor and a screen of the user's smart device where the user has deviated from an ideal position.

### DETAILED DESCRIPTION OF THE FIGURES

As already described, the present application discloses a dynamic posture monitoring system designed to enhance user awareness and encourage proper posture habits and exercise technique. The system may include a belt like apparatus and dual Arduino flex sensors to monitor and notify the wearer about deviation from the preferred posture of the wearer, the bad posture and extended or flexed position of the spine of the wearer that may cause damage to the spine and other parts of the body. The dynamic posture monitoring system of present application is capable of being used in healthcare facilities to help people with back pain, to determine when their spine are in good position. Also, it is capable of being used in the fitness industry to help the individuals to know when their spines are in desired position or in extended position, during the workout.

According to an embodiment, the system of the present application comprises; two Arduino flex sensors to monitor movement of the spine as well as to monitor excess extension and flexion of the spine in real time; a belt with a locking mechanism that can encompass and support sensors and all other modules of the dynamic posture monitoring system and can be wrapped over the stomach and back of the user in such a way that the Arduino flex sensors may come in contact with the spine of the wearer; an electronic control unit working as a brain of the dynamic posture monitoring system, that based on the received data from the sensors, determines the poor position of the spine of the wearer and notifies the wearer physically using vibration as well as sending notification over the smart monitoring device of the wearer through a mobile application; a vibration plate or sensor configured to vibrate and notify the wearer about the poor position of the spine; a mobile application for real time visualization of user's spinal position and to allow re-calibration of the sensor data depending on the user's desired spinal position; a Bluetooth module configured to allow the flex sensors to communicate with the mobile application within the smart device of the user to send notification of poor position of the spine over the smart device such as smart phone or smart watch and also enabling the users to visualize their back position in virtual reality by connecting with the virtual reality headset of the user.

According to another embodiment, the system may use a re-calibration feature where the user instead of an ideal position, can reset the sensor ball to the center of the graph, defining their new "Best position." This position can be different from user to user and is determined based on user's personal needs. Simultaneously, flexion and extension barriers return to predetermined distances from this desired position upon selecting the "Re-calibrate" option in the mobile application. This ensures the sensor ball is always cantered, irrespective of the absolute back position, offering a dynamic and user-centric approach to posture monitoring.

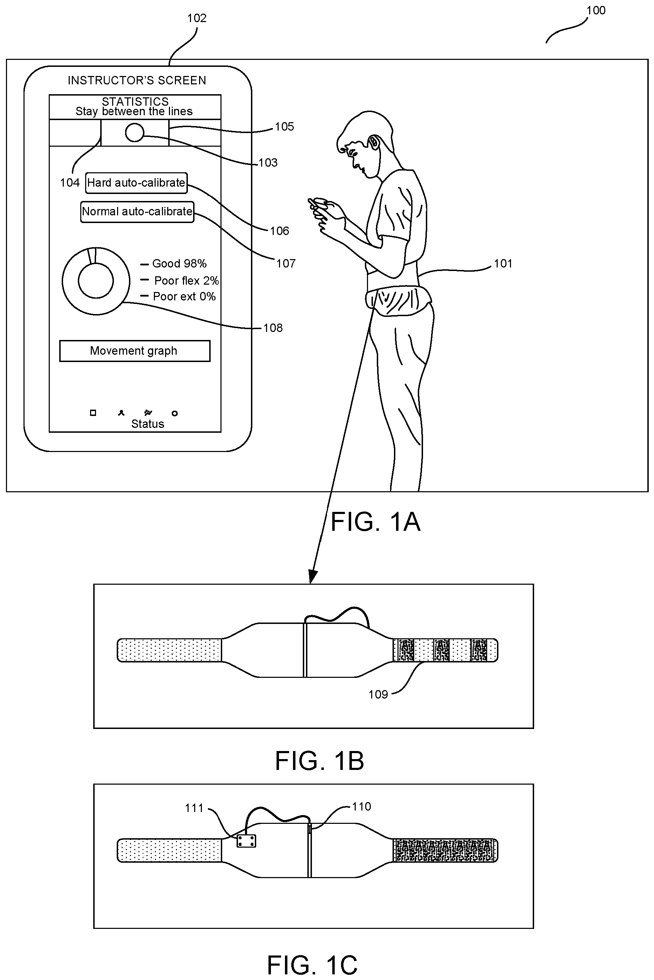

Now referring to **Fig. 1A** which discloses one exemplary embodiment of dynamic posture monitoring system 100 of the present application wherein a user is standing on his feet, thus, having a straight spinal position.

The user wears a belt 101 over their stomach and back, serving as a body attachment means. **Fig. 1B** shows the front view of the user's belt 101, while **Fig. 1C** shows the back view. The belt is worn around the user's waist and uses Velcro 109 as a fastening/locking mechanism, creating a structure that conforms to the spine of any user. Position sensors 110 and a control unit 111 are integrated into the belt 101, with a vibration actuator configured to notify and alert the user of poor spinal posture by vibrating the belt 101. The electronic control unit 111 is preferably connected to a plurality of spine position measuring sensors 110, including flex sensors, strain sensors, or any other position and displacement measuring sensors capable of recording flexion, extension, and movement in the spine. These spinal posture monitoring sensors 110, preferably Arduino flex sensors, are positioned to optimally detect and measure movement, flexion, and extension of the wearer's spine, ideally placed along the user's spine to provide real-time accurate posture data when the belt 101 is worn over the user's stomach and back.

The system 100 preferably also includes a mobile application that displays back or joint position information using a sensor ball 103 on a screen 102. Upper and lower limit flexion 104 and extension 105 barriers are shown as lines to the left and right of the sensor ball 103, alerting the user to excessive movement

The control unit 111 comprises, in addition to a data processing unit and a vibration actuator, a Bluetooth module configured to communicate with the smart device having the mobile application for real-time visualisation of the user's spinal position. The mobile application also allows the position sensors 110 to be recalibrated according to the user's desired spinal position.

In **Fig. 1A****,** the user is standing in an ideal position, with the sensor ball 103 placed in the center of the graph corresponding to the user's spinal position. The flexion line 104 and extension line 105 are positioned to denote the upper and lower limits of the user's spinal position on the graph. A movement graph 108, displayed as a pie chart on the user's smart device screen, shows the user's spinal position as percentage data. For example, when the user is standing straight, which is an ideal position, the percentage data on the movement graph might read 'Good 98%, Poor Flex 2%, Poor Ext 0%.' Based on this data, the user can determine good and bad spinal positions and modify their posture accordingly.

Additionally, the system includes 'Hard Auto Calibrate' and 'Normal Auto Calibrate' functions. These allow the user to calibrate their belt to their ideal position. The user gets into their desired position and then presses either 'Hard' or 'Normal' Auto Calibrate. These functions differ in the amount of spinal movement allowed before the sensor detects misalignment and sends a warning via vibration and visual alert

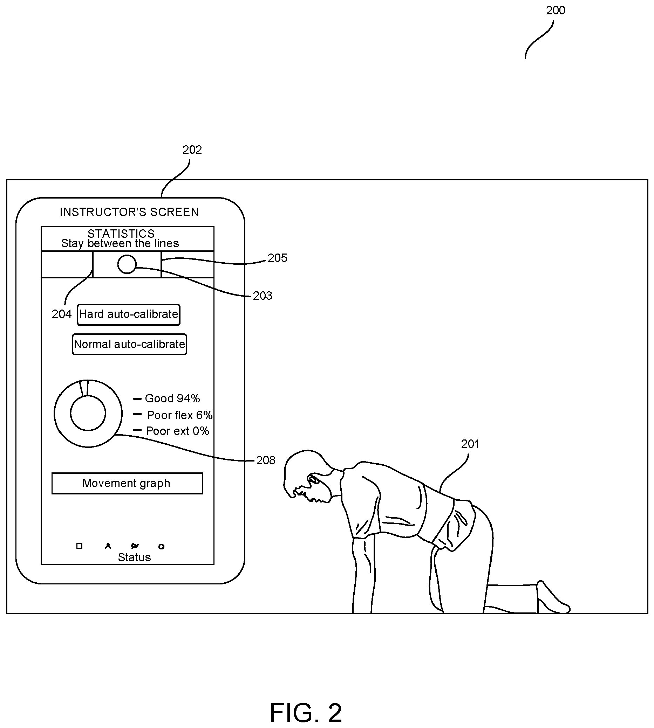

Now referring to **Fig. 2****,** which discloses another exemplary embodiment of the dynamic posture monitoring system 200, showing a user with hands and knees on the floor and a screen 202 of the user's smart device.

In response to user's current position, the Arduino sensors integrated within the belt 201 would measure excessive flex and extension of the user's spine in real time and communicate the data via Bluetooth over mobile application (screen 202 of the smart device shown) if the ideal position settings were still the same as in the **Fig. 1A** embodiment. The sensor ball 203 in the mobile application on the screen 202 would not be in the center of the graph as the position would be different from the ideal position initially defined.

However, since the proposed system 200 allows for recalibration, the settings for the ideal position of the user's spinal posture can be adjusted. This ensures that an ideal back position can be achieved even if the user is not standing or performing other exercises. During recalibration, the flexion and extension barriers, represented by vertical lines 204 and 205, can be reset at a predefined distance from the digital sensor ball 203, which has been moved to the new optimal position by the user. This new optimal position then becomes the new baseline for the ideal posture.

Based on the user's position in the current scenario, the percentage data of the movement graph 208 may also change, for example, now the percentage data would be "Good 94%, Poor Flex 6%, Poor Ext. 0%. This data may differ from user to user depending on their "Best/Desired Position".

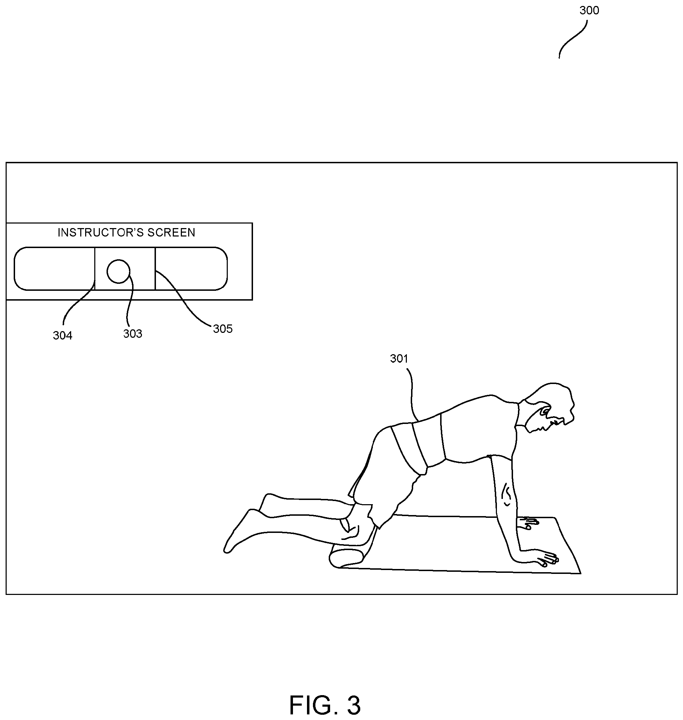

Now referring to **Fig. 3****,** which is another exemplary embodiment of the dynamic posture monitoring system 300, the user's spinal position differs from the previous two examples. The sensor ball 303 is not centered between the flexion and extension barriers, represented by vertical lines 304 and 305, indicating that the user's spinal position is not ideal. However, since the sensor ball 303 has not crossed the lines, no notification or alert is triggered.

If the posture were ideal, the user could recalibrate the flexion and extension barriers, represented by vertical lines 304 and 305. During recalibration, the vertical lines 304 and 305 would shift in relation to the sensor ball 303 to a predefined distance, ensuring that the sensor ball is centered between the lines, thereby marking the new ideal position.

The recalibration is initiated through the user's smart device, which is not shown here in **Fig. 3****.**

### REFERENCE NUMERAL LIST

- **100, 200, 300**: dynamic posture monitoring system
- **101, 201, 301**: belt
- **102, 202**: screen of user's smart device
- **103. 203, 303**: sensor ball
- **104. 204, 304**: flexion line (upper limit)
- **105. 205, 305**: extension line (lower limit)
- **108. 208**: movement graph
- **109**: Velcro
- **110**: spinal posture monitoring sensors
- **111**: electronic control unit

## Claims

1. A dynamic posture monitoring system (100, 200, 300), comprising;
a. a smart device;
b. a plurality of position sensors (110) configured to monitor extension, flexion and movement of a user's spine in real time;
c. an electronic control unit (111) comprising of,
- a data processing unit, a vibration actuator and a Bluetooth module, the data processing unit being in data communication with the position sensors (110), the vibration actuator and the Bluetooth module;
- wherein the data processing unit is configured to receive the monitored extension, flexion and movement of a user's spine from the position sensors (110) as input data, to process this input data to detect a poor position of the user's spine, and to transmit output data to the Bluetooth module and/or as a control signal to the vibration actuator;
- wherein the Bluetooth module is configured to communicate with the smart device having a mobile application for real time visualization of user's spinal position, the mobile application further configured to enable execution of a re-calibration of the position sensors (110) depending on the user's desired spinal position, by transmitting a re-calibration request to the data processing unit, and wherein the system is configured to automatically change the user's desired spinal position during an exercise in response to a selection, by the user via the mobile application, of a program before starting the exercise;
d. a belt (101) for the user's waist, wherein the position sensors (110) and the control unit (111) are integrated into the belt (101), wherein the vibration actuator is configured to notify and alert the user of the poor position of the spine by vibrating the belt (101).

2. The dynamic posture monitoring system (100, 200, 300) according to claim 1, wherein the plurality of position sensors (110) comprises a flex sensor, a strain sensor or any other spine position and displacement measuring sensor.

3. The dynamic posture monitoring system (100, 200, 300) according to any of the preceding claims, wherein the position sensors (110) are integrated into the belt (101) in a way that the position sensors (110) come in contact with the spine of the user.

4. The dynamic posture monitoring system (100, 200, 300) according to any of the preceding claims, wherein the data processing unit is configured to receive data from the position sensors (110), to determine a poor position of the user's spine and to alert the user via the vibration actuator and by sending a notification over the mobile application within the smart device of the user.

5. The dynamic posture monitoring system (100, 200, 300) according to any of the preceding claims, wherein the mobile application is configured to display a spine position through a digital sensor ball (103, 203, 303) and digital flexion and extension barriers.

6. The dynamic posture monitoring system (100, 200, 300) according to claim 5, wherein the flexion and extension barriers are upper and lower limits (104, 204, 304, 105, 205, 305) respectively alerting the user of excessive movement.

7. The dynamic posture monitoring system (100, 200, 300) according to claim 5 or claim 6, wherein the flexion and extension barriers return to predetermined distances from the desired position upon selecting the re-calibration.

8. The dynamic posture monitoring system (100, 200, 300) according to any of the preceding claims, comprising a virtual reality headset connected to the smart device so that the user can visualise the real time spinal movement in virtual reality.

9. A method of monitoring and displaying user's spinal position, the method comprising;
a. providing a dynamic posture monitoring system (100, 200, 300) according to any of the preceding claims;
b. wearing of the belt (101) by a user,
c. acquiring extension, flexion and movement of the user's spine in real time by the position sensor (110);
d. detecting a poor position of the user's spine by the electronic control unit (111);
e. communicating the acquired data of the position sensors (110) and/or the detection of the user's poor spine position to the smart device of the user by the Bluetooth module;
f. visualising of the user's spinal position through the mobile application on the smart device and providing vibrations to the user through the vibration actuator.

10. The method according to claim 9, wherein the method further comprises the step of re-calibrating the position sensors (110) depending on the user's desired spinal position.

11. The method according to claim 10, wherein the re-calibration resets a digital sensor ball (103, 203, 303) to centre of a digital graph based on a user's ideal position.

12. The method according to claim 11, wherein based on the re-caliberated position, digital flexion and extension barriers of the mobile application return to a predetermined distance from the desired position.

## Patentansprüche

1. Dynamisches Haltungsüberwachungssystem (100, 200, 300), umfassend:
a. ein intelligentes Gerät;
b. eine Mehrzahl von Positionssensoren (110), die dazu konfiguriert sind, Extension, Flexion und Bewegung der Wirbelsäule eines Benutzers in Echtzeit zu überwachen;
c. eine elektronische Steuereinheit (111), umfassend:
- eine Datenverarbeitungseinheit, einen Vibrationsaktor und ein Bluetooth-Modul, wobei die Datenverarbeitungseinheit mit den Positionssensoren (110), dem Vibrationsaktor und dem Bluetooth-Modul in Datenkommunikation steht;
- wobei die Datenverarbeitungseinheit dazu konfiguriert ist, die überwachte Extension, Flexion und Bewegung der Wirbelsäule eines Benutzers von den Positionssensoren (110) als Eingangsdaten zu empfangen, diese Eingangsdaten zu verarbeiten, um eine schlechte Stellung der Wirbelsäule des Benutzers zu erkennen, und Ausgangsdaten an das Bluetooth-Modul und/oder als Steuersignal an den Vibrationsaktor zu übertragen;
- wobei das Bluetooth-Modul dazu konfiguriert ist, mit dem intelligenten Gerät zu kommunizieren, das eine mobile Anwendung zur Echtzeitvisualisierung der Wirbelsäulenstellung des Benutzers aufweist, wobei die mobile Anwendung ferner dazu konfiguriert ist, die Ausführung einer Neukalibrierung der Positionssensoren (110) in Abhängigkeit von der gewünschten Wirbelsäulenstellung des Benutzers zu ermöglichen, indem sie eine Neukalibrierungsanforderung an die Datenverarbeitungseinheit überträgt, und wobei das System dazu konfiguriert ist, die gewünschte Wirbelsäulenstellung des Benutzers während einer Übung als Reaktion auf eine vor Beginn der Übung erfolgende Auswahl eines Programms durch den Benutzer über die mobile Anwendung automatisch zu ändern;
d. einen Gürtel (101) für die Taille des Benutzers, wobei die Positionssensoren (110) und die Steuereinheit (111) in den Gürtel (101) integriert sind, wobei der Vibrationsaktor dazu konfiguriert ist, den Benutzer durch Vibrieren des Gürtels (101) über die schlechte Stellung der Wirbelsäule zu benachrichtigen und zu warnen.

2. Dynamisches Haltungsüberwachungssystem (100, 200, 300) nach Anspruch 1, wobei die Mehrzahl von Positionssensoren (110) einen Biegesensor, einen Dehnungssensor oder einen beliebigen anderen Sensor zur Messung der Stellung und Verschiebung der Wirbelsäule umfasst.

3. Dynamisches Haltungsüberwachungssystem (100, 200, 300) nach einem der vorhergehenden Ansprüche, wobei die Positionssensoren (110) derart in den Gürtel (101) integriert sind, dass die Positionssensoren (110) mit der Wirbelsäule des Benutzers in Kontakt kommen.

4. Dynamisches Haltungsüberwachungssystem (100, 200, 300) nach einem der vorhergehenden Ansprüche, wobei die Datenverarbeitungseinheit dazu konfiguriert ist, Daten von den Positionssensoren (110) zu empfangen, eine schlechte Stellung der Wirbelsäule des Benutzers zu bestimmen und den Benutzer über den Vibrationsaktor sowie durch Senden einer Benachrichtigung über die mobile Anwendung auf dem intelligenten Gerät des Benutzers zu warnen.

5. Dynamisches Haltungsüberwachungssystem (100, 200, 300) nach einem der vorhergehenden Ansprüche, wobei die mobile Anwendung dazu konfiguriert ist, eine Wirbelsäulenstellung mittels einer digitalen Sensorkugel (103, 203, 303) und digitaler Flexions- und Extensionsbarrieren anzuzeigen.

6. Dynamisches Haltungsüberwachungssystem (100, 200, 300) nach Anspruch 5, wobei die Flexions- und Extensionsbarrieren obere bzw. untere Grenzen (104, 204, 304, 105, 205, 305) sind, die den Benutzer vor übermäßiger Bewegung warnen.

7. Dynamisches Haltungsüberwachungssystem (100, 200, 300) nach Anspruch 5 oder Anspruch 6, wobei die Flexions- und Extensionsbarrieren bei Auswahl der Neukalibrierung auf vorbestimmte Abstände von der gewünschten Stellung zurückkehren.

8. Dynamisches Haltungsüberwachungssystem (100, 200, 300) nach einem der vorhergehenden Ansprüche, umfassend ein Virtual-Reality-Headset, das mit dem intelligenten Gerät verbunden ist, sodass der Benutzer die Echtzeitbewegung der Wirbelsäule in virtueller Realität visualisieren kann.

9. Verfahren zum Überwachen und Anzeigen der Wirbelsäulenstellung eines Benutzers, wobei das Verfahren umfasst:
a. Bereitstellen eines dynamischen Haltungsüberwachungssystems (100, 200, 300) nach einem der vorhergehenden Ansprüche;
b. Tragen des Gürtels (101) durch einen Benutzer;
c. Erfassen von Extension, Flexion und Bewegung der Wirbelsäule des Benutzers in Echtzeit durch den Positionssensor (110);
d. Erkennen einer schlechten Stellung der Wirbelsäule des Benutzers durch die elektronische Steuereinheit (111);
e. Übermitteln der erfassten Daten der Positionssensoren (110) und/oder der Erkennung der schlechten Wirbelsäulenstellung des Benutzers an das intelligente Gerät des Benutzers durch das Bluetooth-Modul;
f. Visualisieren der Wirbelsäulenstellung des Benutzers über die mobile Anwendung auf dem intelligenten Gerät und Bereitstellen von Vibrationen an den Benutzer durch den Vibrationsaktor.

10. Verfahren nach Anspruch 9, wobei das Verfahren ferner den Schritt des Neukalibrierens der Positionssensoren (110) in Abhängigkeit von der gewünschten Wirbelsäulenstellung des Benutzers umfasst.

11. Verfahren nach Anspruch 10, wobei die Neukalibrierung eine digitale Sensorkugel (103, 203, 303) basierend auf einer idealen Stellung eines Benutzers auf die Mitte eines digitalen Graphen zurücksetzt.

12. Verfahren nach Anspruch 11, wobei basierend auf der neukalibrierten Stellung digitale Flexions- und Extensionsbarrieren der mobilen Anwendung auf einen vorbestimmten Abstand von der gewünschten Stellung zurückkehren.

## Revendications

1. Système dynamique de surveillance de la posture (100, 200, 300), comprenant :
a. un appareil intelligent ;
b. une pluralité de capteurs de position (110) configurés pour surveiller l'extension, la flexion et le mouvement de la colonne vertébrale d'un utilisateur en temps réel ;
c. une unité de commande électronique (111) comprenant :
- une unité de traitement de données, un actionneur de vibration et un module Bluetooth, l'unité de traitement de données étant en communication de données avec les capteurs de position (110), l'actionneur de vibration et le module Bluetooth ;
- dans lequel l'unité de traitement de données est configurée pour recevoir des capteurs de position (110), en tant que données d'entrée, l'extension, la flexion et le mouvement surveillés de la colonne vertébrale d'un utilisateur, pour traiter ces données d'entrée afin de détecter une mauvaise position de la colonne vertébrale de l'utilisateur, et pour transmettre des données de sortie au module Bluetooth et/ou sous forme de signal de commande à l'actionneur de vibration ;
- dans lequel le module Bluetooth est configuré pour communiquer avec l'appareil intelligent comportant une application mobile pour la visualisation en temps réel de la position de la colonne vertébrale de l'utilisateur, l'application mobile étant en outre configurée pour permettre l'exécution d'un recalibrage des capteurs de position (110) en fonction de la position de la colonne vertébrale souhaitée par l'utilisateur, en transmettant une demande de recalibrage à l'unité de traitement de données, et dans lequel le système est configuré pour modifier automatiquement la position de la colonne vertébrale souhaitée par l'utilisateur au cours d'un exercice en réponse à une sélection d'un programme, par l'utilisateur via l'application mobile, avant le début de l'exercice ;
d. une ceinture (101) destinée à la taille de l'utilisateur, dans lequel les capteurs de position (110) et l'unité de commande (111) sont intégrés dans la ceinture (101), dans lequel l'actionneur de vibration est configuré pour notifier et alerter l'utilisateur de la mauvaise position de la colonne vertébrale en faisant vibrer la ceinture (101).

2. Système dynamique de surveillance de la posture (100, 200, 300) selon la revendication 1, dans lequel la pluralité de capteurs de position (110) comprend un capteur de flexion, un capteur de déformation ou tout autre capteur de mesure de la position et du déplacement de la colonne vertébrale.

3. Système dynamique de surveillance de la posture (100, 200, 300) selon l'une quelconque des revendications précédentes, dans lequel les capteurs de position (110) sont intégrés dans la ceinture (101) de manière à ce que les capteurs de position (110) entrent en contact avec la colonne vertébrale de l'utilisateur.

4. Système dynamique de surveillance de la posture (100, 200, 300) selon l'une quelconque des revendications précédentes, dans lequel l'unité de traitement de données est configurée pour recevoir des données des capteurs de position (110), pour déterminer une mauvaise position de la colonne vertébrale de l'utilisateur et pour alerter l'utilisateur via l'actionneur de vibration et en envoyant une notification par l'intermédiaire de l'application mobile sur l'appareil intelligent de l'utilisateur.

5. Système dynamique de surveillance de la posture (100, 200, 300) selon l'une quelconque des revendications précédentes, dans lequel l'application mobile est configurée pour afficher une position de la colonne vertébrale au moyen d'une boule de capteur numérique (103, 203, 303) et de barrières numériques de flexion et d'extension.

6. Système dynamique de surveillance de la posture (100, 200, 300) selon la revendication 5, dans lequel les barrières de flexion et d'extension sont respectivement des limites supérieure et inférieure (104, 204, 304, 105, 205, 305) alertant l'utilisateur d'un mouvement excessif.

7. Système dynamique de surveillance de la posture (100, 200, 300) selon la revendication 5 ou la revendication 6, dans lequel les barrières de flexion et d'extension reviennent à des distances prédéterminées de la position souhaitée lors de la sélection du recalibrage.

8. Système dynamique de surveillance de la posture (100, 200, 300) selon l'une quelconque des revendications précédentes, comprenant un casque de réalité virtuelle connecté à l'appareil intelligent de sorte que l'utilisateur puisse visualiser en réalité virtuelle le mouvement de la colonne vertébrale en temps réel.

9. Procédé de surveillance et d'affichage de la position de la colonne vertébrale d'un utilisateur, le procédé comprenant :
a. la fourniture d'un système dynamique de surveillance de la posture (100, 200, 300) selon l'une quelconque des revendications précédentes ;
b. le port de la ceinture (101) par un utilisateur ;
c. l'acquisition de l'extension, de la flexion et du mouvement de la colonne vertébrale de l'utilisateur en temps réel par le capteur de position (110) ;
d. la détection d'une mauvaise position de la colonne vertébrale de l'utilisateur par l'unité de commande électronique (111) ;
e. la communication des données acquises des capteurs de position (110) et/ou de la détection de la mauvaise position de la colonne vertébrale de l'utilisateur à l'appareil intelligent de l'utilisateur par le module Bluetooth ;
f. la visualisation de la position de la colonne vertébrale de l'utilisateur au moyen de l'application mobile sur l'appareil intelligent et la fourniture de vibrations à l'utilisateur par l'actionneur de vibration.

10. Procédé selon la revendication 9, dans lequel le procédé comprend en outre l'étape de recalibrage des capteurs de position (110) en fonction de la position de la colonne vertébrale souhaitée par l'utilisateur.

11. Procédé selon la revendication 10, dans lequel le recalibrage réinitialise une boule de capteur numérique (103, 203, 303) au centre d'un graphique numérique sur la base d'une position idéale d'un utilisateur.

12. Procédé selon la revendication 11, dans lequel, sur la base de la position recalibrée, des barrières numériques de flexion et d'extension de l'application mobile reviennent à une distance prédéterminée de la position souhaitée.
